# EUROPÄISCHE PATENTSCHRIFT

(11) **EP 1 393 766 B1**
(45) Veröffentlichungstag und Bekanntmachung des Hinweises auf die Patenterteilung: **07.01.2009**
(21) Anmeldenummer: 03090242.3
(22) Anmeldetag: 31.07.2003
(51) Int. Cl.: A61M 5/32, A61M 25/00, A61F 2/06

(54) **Endovaskuläres Implantat zur Injektion eines Wirkstoffs in die Media eines Blutgefässes**
Endovascular implant for injecting a drug into the media of a blood vessel
Implant endovasculaire pour injecter un principe actif dans le media d'un vaisseau sanguin

(30) Priorität: 31.07.2002 DE 10235689
(43) Veröffentlichungstag der Anmeldung: 03.03.2004
(73) Patentinhaber: BIOTRONIK GmbH & Co. KG, 12359 Berlin (DE)
(72) Erfinder: Momma, Carsten, 18055 Rostock (DE); Becker, Andreas, 10827 Berlin (DE); Schmiedel, Robert, 96049 Bamberg (DE); Heublein, Bernd, 30627 Hannover (DE)
(74) Vertreter: Lindner-Vogt, Karin L.

(56) Entgegenhaltungen:
- WO-A-00/67647
- WO-A-01/49357
- US-B1- 6 210 392
- US-B1- 6 254 632
- US-B1- 6 283 947
- US-B1- 6 503 231
- RANINEN REINO O: "Ultrasound imaging for arterial wall thickness measurement: An in vitro study with stereomicroscopic correlation" ULTRASOUND MED BIOL;ULTRASOUND IN MEDICINE AND BIOLOGY JUL 1998 ELSEVIER SCI LTD, EXETER, ENGL, Bd. 24, Nr. 6, Juli 1998 (1998-07), Seiten 833-839, XP002258785

## Beschreibung

Die Erfindung betrifft ein endovaskuläres Implantat zur Applikation eines Wirkstoffs in die Media eines Blutgefäßes nach Anspruch 1 sowie ein Verfahren zur Herstellung eines derartigen Implantats nach den Anspruch 19.

Einer der häufigsten Todesursachen in Westeuropa und Nordamerika sind koronare Herzkrankheiten. Nach neuen Erkenntnissen sind vor allem entzündliche Prozesse treibende Kraft der Arteriosklerose. Initiiert wird der Prozess vermutlich durch die vermehrte Einlagerung von Lipoproteinen geringer Dichte (LDL-Partikel) in die Intima der Gefäßwand. Nach dem Eindringen in die Intima werden die LDL-Partikel durch Oxidantien chemisch modifiziert. Die modifizierten LDL-Partikel veranlassen wiederum die Endothelzellen, die die inneren Gefäßwände auskleiden, das Immunsystem zu aktivieren. In der Folge treten Monocyten in die Intima ein und reifen zu Makrophagen heran. Im Zusammenspiel mit den ebenfalls eintretenden T-Zellen werden Entzündungsmediatoren, wie Immunbotenstoffe und proliferativ wirkende Substanzen, freigesetzt, und die Makrophagen beginnen die modifizierten LDL-Partikel aufzunehmen. Die sich bildenden Lipidläsionen aus T-Zellen und den mit LDL-Partikeln gefüllten Makrophagen, die aufgrund ihres Aussehens Schaumzellen genannt werden, stellen eine Frühform der arteriosklerotischen Plaque dar. Die Entzündungsreaktion in der Intima veranlasst durch entsprechende Entzündungsmediatoren glatte Muskelzellen der weiter außen liegenden Media der Gefäßwand bis unter die Endothelzellen zu wandern. Dort vermehren sie sich und bilden eine fibröse Deckschicht aus dem Faserprotein Kollagen, die den darunter liegenden Lipidkern aus Schaumzellen gegen die Blutbahn abgrenzt. Die dann vorliegenden tiefgreifenden strukturellen Veränderungen in der Gefäßwand werden zusammenfassend als Plaque bezeichnet.

Der arteriosklerotische Plaque expandiert zunächst relativ wenig in Richtung der Blutbahn, da diese sich kompensatorisch erweitern kann. Mit der Zeit kommt es jedoch zu einer Verengung des Blutkanals (Stenose), deren erste Anzeichen bei körperlicher Belastung auftreten. Die verengte Arterie kann sich dann nicht mehr ausreichend weiten, um das zu versorgenden Gewebe besser zu durchbluten. Ist eine Herzarterie betroffen, so klagt der Patient häufig über ein Druck- und Engegefühl hinter dem Brustbein (Angina pectoris). Bei anderen Arterien sind schmerzhafte Krämpfe ein häufig auftretendes Indiz für die Stenose.

Die Stenose kann letztendlich zu einem völligen Verschließen der Blutbahn führen (Herzinfarkt, Schlaganfall). Allein durch die Plaquebildung tritt dies nach neueren Untersuchungen jedoch nur in etwa 15 Prozent der Fälle auf. Vielmehr scheint der durch bestimmte Entzündungsmediatoren aus den Schaumzellen veranlasste allmähliche Abbau der fibrösen Deckschicht aus Kollagen ein entscheidender Zusatzfaktor zu sein. Reißt die fibröse Deckschicht auf, so kann der Lipidkern unmittelbar mit dem Blut in Kontakt kommen. Da in Folge der Entzündungsreaktion gleichzeitig Gewebsfaktoren (TF tissue factor) in den Schaumzellen produziert werden, die sehr potente Auslöser der Gerinnungskaskade sind, kann der sich bildende Blutpfropf das Blutgefäß akut vollständig (akuter Infarkt) oder teilweise (instabile Angina pectoris) verschließen.

Seit mehr als zwanzig Jahren sind nicht-operative Methoden zur Stenose-Behandlung etabliert, bei denen u.a. durch Ballondilatation (PTCA Perkutane Transluminale Coronare Angioplastie) das Blutgefäß wieder aufgeweitet wird. Mit dem Aufweiten des Blutgefäßes entstehen allerdings Verletzungen (Einrisse, sog. Dissektionen) in der Gefäßwand, die zwar überwiegend problemlos verheilen, jedoch in etwa einem Drittel der Fälle durch das ausgelöste Zellwachstum zu Wucherungen führen (Proliferation), die letztendlich zu einer erneuten Gefäßverengung (Restenose) führen. Die Aufweitung beseitigt auch nicht die physiologischen Ursachen der Stenose, also die Veränderungen in der Gefäßwand. Eine weitere Ursache der Restenose ist die Elastizität des gedehnten Blutgefäßes. Nach dem Entfernen des Ballons zieht sich das Blutgefäß übermäßig zusammen, so dass der Gefäßquerschnitt verringert wird (Obstruktion, sog. Negatives remodelling). Letzterer Effekt kann nur durch Platzierung eines Stents vermieden werden. Durch den Einsatz von Stents kann zwar ein optimaler Gefäßquerschnitt erreicht werden, allerdings führt der Einsatz von Stents ebenfalls zu kleinsten Verletzungen, die die Proliferation induzieren können und damit letztendlich eine Restenose auslösen können.

Mittlerweile bestehen umfangreiche Erkenntnisse zum zellbiologischen Mechanismus und zu den auslösenden Faktoren der Stenose und Restenose. Die Restenose entsteht - wie bereits erläutert - als Reaktion der Gefäßwand auf die lokale Verletzung infolge Dehnung des artherosklerotischen Plaque. Über komplexe Wirkmechanismen wird die lumengerichtete Migration und Proliferation der glatten Muskelzellen der Media und der Adventitia induziert (neointimale Hyperplasie). Unter Einfluss verschiedener Wachstumsfaktoren produzieren die glatten Muskelzellen eine Deckschicht aus Matrixproteinen (Elastin, Kollagen, Proteoglykane), deren ungesteuertes Wachstum allmählich zu einer Einengung des Lumens führen kann. Systematisch-medikamentöse Therapieeinsätze sehen u.a. die orale Verabreichung von Calzium-Antagonisten, ACE-Hemmern, Antikoagulantien, Antiaggregantien, Fischölen, antiproliferativen Substanzen, antiinflammatorischen Substanzen und Serotonin-Antagonisten vor, signifikante Reduktionen der Restenoseraten wurden jedoch bisher nicht erreicht.

Ein Grundproblem in der medikamentösen Behandlung bzw. Prävention der Restenose sind die teils erheblichen Nebenwirkungen der Wirkstoffe. Diese versucht man unter anderem durch eine möglichst lokal begrenzte Applikation in entsprechend geringen Dosen zu umgehen. Das sogenannte Konzept des Local Drug Delivery (LDD) sieht vor, den oder die Wirkstoffe unmittelbar am Ort des Geschehens und begrenzt auf dieses Areal freizusetzen. Dazu wird häufig eine der Gefäßwand zugewandte Oberfläche des endovaskulären Implantats, also insbesondere eines Stents, mit einer aktiven Beschichtung versehen. Die aktive Komponente der Beschichtung in Form eines therapeutischen Wirkstoff, kann direkt an der Oberfläche des Implantats gebunden oder in einem geeigneten Arzneistoffträger eingebettet sein. Im letzteren Fall wird durch Diffusion und gegebenenfalls allmählichen Abbau des biodegradierbaren Trägers der Wirkstoff freigesetzt.

In der US 6,287,628 wird ein Implantat und ein Verfahren zu dessen Herstellung vorgeschlagen, bei dem der Wirkstoff nicht direkt auf der Oberfläche des Implantats aufgebracht, sondern in einem Wirkstoffdepot bereitgestellt wird. Die Wirkstoffdepots sind als Kavitäten im Grundkörper des Implantats eingebracht und auf der Außenseite des Implantats angeordnet. Hierdurch soll ein übermäßiges Ausspülen des Wirkstoff durch den immerwährenden Blutstrom verhindert werden.

Ferner zeigt die US 6,254,632 Strukturen, die aus der Ebene der Implantatsoberfläche herausragen und die u.a. zur Bereitstellung des Wirkstoffs dienen sollen. Die kraterförmigen Strukturen sollen die Abgabe der Substanz direkt an die Gefäßwand unterstützen. Form und insbesondere Höhe der Struktur - diese liegen den Angaben nach im Bereich von 10 bis 80 µm - werden so vorgegeben, dass die Struktur nicht in die Gefäßwand eindringt, sondern diese allenfalls verformt.

Bei allen bekannten Lösungen liegt die Oberfläche des Implantats direkt an der Intima des Blutgefäßes an. Der freigesetzte Wirkstoff muss demnach durch Diffusion zunächst diese Grenzschicht passieren. Die Diffusion des Wirkstoffs kann allerdings durch in der Regel vorhandene Plaque, Kalzifizierung oder verstärkte Gefäßwandschichten behindert werden. Gerade bei koronaren Erkrankungen haben Studien belegt, dass die Intima in ihrer Wanddicke deutlich verstärkt ist und über 150 µm breit sein kann. Darüber hinaus können erhebliche Mengen des Wirkstoffs durch den ständig währenden Lumenfluss im Blutgefäß mitgerissen werden, solange der Wirkstoff nicht in die Gefäßwand eingedrungen ist. Für bestimmte medikamentöstherapeutischen Wirkungen (z.B. Gen-Therapie) ist es erforderlich, möglichst schnell und mit hoher Konzentration an oder in die Nähe der glatten Muskelzellen der Gefäßwand (Media-Region) zu kommen.

Der vorliegenden Erfindung liegt daher die Aufgabe zugrunde ein endovaskuläres Implantat zu schaffen, dass die geschilderten Nachteile des Standes der Technik überwindet und eine Applikation kleinster Wirkstoffmengen in einem lokal begrenzten Bereich, insbesondere direkt in die Media der Gefäßwand, ermöglicht.

Die Aufgabe wird durch das endovaskuläre Implantat zur Applikation eines Wirkstoffs in die Media eines Blutgefäßes mit den im Anspruch 1 genannten Merkmalen und die dazugehörigen Herstellungsverfahren nach den Ansprüchen 20 und 21 gelöst. Der erfindungsgemäße Grundkörper des Implantats weist dazu zumindest abschnittsweise an einer der Gefäßwand zugewandten Oberfläche eine Vielzahl von Mikrovorrichtungen zur Applikation des Wirkstoffs auf. Jede Mikrovorrichtung umfasst
- zumindest eine Mikrokanüle, die soweit aus der Ebene der Oberfläche des Implantats herausgehoben ist, dass sie beim flächigen Anlegen des Implantats an die Gefäßwand des Blutgefäßes bis in die Media des Blutgefäßes eindringt und
- zumindest ein Wirkstoffdepot, dass mit zumindest einer Mikrokanüle in Verbindung steht.

Mit der vorgenannten erfindungsgemäßen Ausgestaltung des Implantats lassen sich die Wirkstoffe direkt am spezifischen Ort ihres Wirkens, nämlich der Media, eluieren. Damit einhergehend kann die applizierte Wirkstoffmenge wesentlich verringert werden, so dass die Herstellungskosten des LDD-Implantats deutlich gesenkt werden können und alokale Nebenwirkungen weitestgehend ausgeschlossen sind.

Gemäß der Erfindung ragen die Mikrokanülen etwa 100-400 µm aus der Oberfläche des Implantats heraus. So ist er sichergestellt, dass sie die Intima durchstoßen, aber noch nicht tieferliegende Gefäßbereiche, d. h. die Adventitia, erreichen. Beim intrakoronaren Einsatz sind aufgrund der anatomischen Besonderheiten der Gefäßwände Mikrokanülenlängen von 150-300 µm, insbesondere 180-250 µm, bevorzugt, da sie in den allermeisten Fällen eine optimale Eindringtiefe der Mikrokanülen bieten. Die Dicke der Gefäßwände kann im Einzelfall allerdings deutlich abweichen, denn neben den krankheitsbedingten Faktoren sind individuelle Variationen der Gefäßwandbreiten belegt. Mittlerweile wurden Diagnostiksysteme entwickelt (z. B. intravaskulärer Ultraschall), die eine Messung der Dicke der Intima erlauben, so dass dem behandelnden Arzt ein zusätzliches diagnostisches Instrumentarium für die Wahl des Implantats mit einer ausreichend dimensionierten Mikrokanülenlänge bereit steht.

Die Mikrovorrichtung kann zum einen integraler Bestandteil des Grundkörpers des Implantats sein, also durch geeignete Bearbeitungsschritte aus diesem geformt werden. Zum anderen kann die Mikrovorrichtung aber auch als auf dem Grundkörper aufgesetzte Struktur realisiert werden (Hybrid-Technik).

Im ersteren Fall wird das Implantat vorzugsweise derart hergestellt, dass in den metallischen Grundkörper des Implantats durch partielle Materialabtragung auf der Gefäßwand zugewandten Oberfläche des Implantats eine Kavität eingebracht wird. Dies kann beispielsweise mit Rückgriff auf bewährte Laserbearbeitungsprozesse erfolgen, die zumeist auch beim Herausschneiden der Stentstrukturen angewendet werden. Die einige Mikrometer tiefe Kavität wird in einem anschließenden Prozessschritt mit einem isolierendem Material bedeckt, wobei das Material derart aufgetragen wird, dass es einen umlaufenden Rand der Kavität leicht überlappt. Danach wird durch Elektropolieren in den nicht durch das isolierende Material bedeckten Bereich der Oberfläche das Material des Grundkörpers abgetragen, wobei sich allmählich die gewünschten Mikrokanülen oberhalb der Kavität ausbilden. Nach Beendigung des Elektropolierens wird das isolierende Material mit einem geeigneten Lösungsmittel entfernt. Das Verfahren modifiziert damit lediglich an sich bekannte Bearbeitungsschritte bei der Herstellung von Stents, so dass auf vorhandene Erfahrungsschätze zurückgegriffen werden kann.

Sollen die Mikrovorrichtungen in Hybrid-Technik als unabhängige Strukturen auf der Oberfläche des Implantats realisiert werden, so kann auf an sich bekannte Rapid-Prototyping-Verfahren zur Polymerabscheidung und zum Sintern zurückgegriffen werden. So kann beispielsweise durch Mikro-Stereolithographie unter Einwirkung von Licht eine Verkettung von Monomeren zu Polymeren induziert werden. Eine Laser-Scannereinheit belichtet dazu schraffurartig eine definierte Fläche auf der Oberfläche des flüssigen Monomers und härtet auf diese Weise mit einer bestimmten Eindringtiefe eine Schicht des zu fertigenden Modells aus. Eine Verstelleinheit in z-Richtung sorgt dafür, dass das Substrat Schicht um Schicht um die definierte Schichtdicke abgesenkt bzw. den Laserfokus hochgefahren wird. Im folgenden Bearbeitungsschritt wird das Monomer über der zuvor erzeugten festen Schicht durch Belichtung zur Polymerisation gebracht und dieser Bearbeitungsschritt wird solange wiederholt bis die gewünschte Struktur steht. Nach der Schichtengenerierung werden verbleibende Monomerreste durch geeignete Lösungsmittel entfernt. Die Prozesse werden durch komplexe Steuermechanismen überwacht und erlauben die Herstellung von Mikrostrukturen mit Mikrometerdimensionen, die zuvor mit gängigen Programmen als z.B. CAD-Vorlage erstellt wurden. Die durch Rapid-Prototyping-Verfahren erzeugten Mikrovorrichtungen beinhalten in jedem Fall ein Wirkstoffdepot, dass mit zumindest einer Mikrokanüle verbunden ist.

In einer Variante der Erfindung wird ein Freisetzungsverhalten des zumindest einen deponierten Wirkstoffs so festgelegt, dass der zumindest eine Wirkstoff erst nach Eindringen der Mikrokanülen in die Media des Blutgefäßes freigesetzt wird. Eine unkontrollierte Eluierung soll damit vermieden und die Gesamtdosis des Wirkstoffs weiter reduziert werden. So kann die Mikrovorrichtung nach Einbringung des zumindest einen Wirkstoffs in das Wirkstoffdepot durch eine Deckschicht aus einem biodegradierbaren Material verschlossen werden, welches erst nach dem Eindringen in die Media vollständig abgebaut ist. Weiterhin ist denkbar, dass der zumindest eine Wirkstoff in einem biodegradierbaren Arzneistoffträger eingebettet ist. Auch nach dieser Variante wird über das Degradationsverhalten des Trägers das Freisetzungsverhalten des Wirkstoffs beeinflusst. Selbstverständlich können beide Lösungsvorschläge miteinander kombiniert werden, d.h. durch eine geeignete Deckschicht und einen Arzneistoffträger wird die Freisetzung im erfindungsgemäßen Sinne kontrolliert.

Ferner ist bevorzugt, dass mehrere Wirkstoffe im Wirkstoffdepot bereitgestellt werden und deren Freisetzung stufenweise erfolgt. Dies kann beispielsweise derart geschehen, dass mehrere Schichten biodegradierbarer Arzneistoffträger mit verschiedenen Wirkstoffen schichtweise in das Wirkstoffdepot eingebracht werden. Von außen nach innen werden die Schichten nacheinander abgebaut und setzen dementsprechend die verschiedenen Wirkstoffe nacheinander frei. Denkbar ist auch, ein oder mehrere Trennschichten - wiederum aus einem biodegradierbaren Material - in das Wirkstoffdepot einzubringen. Die Trennschichten dienen der Separation der verschiedenen Wirkstoffe und werden nacheinander abgebaut. Durch ein derartiges zeitlich abgestuftes Vorgehen können die der Restenose zugrunde liegenden Mechanismen wirkungsvoll beeinflusst werden. So können beispielsweise durch antiproliferative Substanzen die initialen Mechanismen der Restenose gezielt bekämpft werden und zu einem späteren Zeitpunkt durch Applikation von antiinflamatorischen Substanzen und Ähnlichem die Zellenmigration verhindert werden.

Vorzugsweise werden auch die außerhalb der Mikrovorrichtung liegenden Bereiche der Oberfläche des Implantats mit einer Schicht aus einem biodegradierbarem Material bedeckt. Es hat sich nämlich herausgestellt, dass derartige bioaktive Oberflächen die Restenoserate deutlich senken. Bevorzugte Materialien sind Hyaluronsäurepolymer, Polylactide und Heparin.

Wenn die Schicht in Umfangsrichtung bündig mit einer Spitze der Mikrokanüle der Mikrovorrichtung abschließt oder diese gar vollständig bedeckt (covering), so ist die Struktur zum einen zunächst vor mechanischer Beschädigung bei der Einbringung in den Körper geschützt und zum anderen lässt sich das Eindringen der Mikrokanüle in die Gefäßwand zeitlich steuern. Durch den allmählichen Abbau der Schicht dringen die Mikrokanülen langsam durch die Intima in die Media ein. Das langsame Eindringen verhindert Gefäßwandverletzungen, die wiederum Ausgangspunkt für Restenoseprozesse sein könnten. Um eine frühzeitige Freisetzung des Wirkstoffes zu verhindern, muss das Freisetzungsverhalten des deponierten Wirkstoffs durch Wahl des biodegradierbaren Arzneistoffträgers bzw. einer Deckschicht entsprechend abgestimmt werden. Besonders geeignet für die Beschichtung der Oberfläche in den außerhalb der Mikrovorrichtung liegenden Bereichen ist Hyaluronsäure. Ein Abbauverhalten der Hyaluronsäure kann durch gezielte Vernetzung in der gewünschten Weise festgelegt werden. Daher eignet sich dieses Material auch als Deckschicht und Arzneistoffträger. Die Herstellung vernetzter Hyaluronsäurebeschichtungen und die Beeinflussung des Degradationsverhaltens ist dem Prinzip nach aus dem Stand der Technik bekannt. Im allgemeinen steigt mit steigendem Polymerisationsgrad und/oder Vernetzungsgrad des Trägers die Degradationszeit an. Eine sich für den eingebetteten Wirkstoff ergebende Elutionscharakteristik hängt, neben Diffusionsvorgängen, vom Polymerisations- und Vernetzungsgrad ab. In der Regel wird die Elution mit steigender Degradationszeit verlängert.

Das Implantat ist ein Stent, insbesondere ein Koronarstent, denn gerade bei diesen Implantaten ist die Restenosegefahr groß. Der Stent besitzt zudem vorzugsweise selbstexpandierende Strukturen, die ein allmähliches Eindringen der Mikrokanüle in die Gefäßwand unterstützen. Dies ist besonders wünschenswert, wenn die Mikrovorrichtungen, wie vorab beschrieben, mit einer Schutzschicht (covering) abgedeckt sind. Natürlich ist es auch möglich, das Eindringen der Mikrokanülen durch Ballondilatation zu erreichen.

In einer weiteren Variante der Erfindung ist auch der Grundkörper des Implantats, insbesondere des Stents, aus einer biodegradierbaren Magnesiumlegierung geformt. Der vollständige Abbau des Implantats behebt langfristig die die Restenose möglicherweise auslösenden Faktoren. Weitere, bevorzugte Ausgestaltung der Erfindung ergeben sich aus den übrigen in den Unteransprüchen genannten Merkmalen.

Die Erfindung wird nachfolgend anhand eines Ausführungsbeispiels und in mehreren Zeichnungen näher erläutert. Es zeigen:
- Fig. 1: einen schematischen Querschnitt durch ein endovaskuläres Implantat im Bereich einer Mikrovorrichtung und nach dem Eindringen in eine Gefäßwand;
- Fig. 2 - 5: einen schematischen Querschnitt durch Mikrovorrichtungen, bei denen der Wirkstoff entsprechend vier Alternativen bereitgestellt wird;
- Fig. 6: die Mikrovorrichtung mit einer sie umgebenden Zusatzbeschichtung;
- Fig. 7: die Mikrovorrichtung mit einer sie umgebenden Zusatzbeschichtung als Schutzschicht (covering);
- Fig. 8a - 8d: eine schematische Darstellung eines Herstellungsprozesses der Mikrovorrichtung nach einer ersten Variante;
- Fig. 9: eine Prinzipanordnung zum Rapid-Prototyping-Verfahren, mit dem die Herstellung der Mikrovorrichtung nach einer zweiten Variante durchführbar ist und
- Fig. 10a-10b: eine schematische Darstellung zweier Mikrovorrichtung, die nach dem Rapid-Prototyping-Verfahren auf einer Implantatsoberfläche erzeugt wurden.

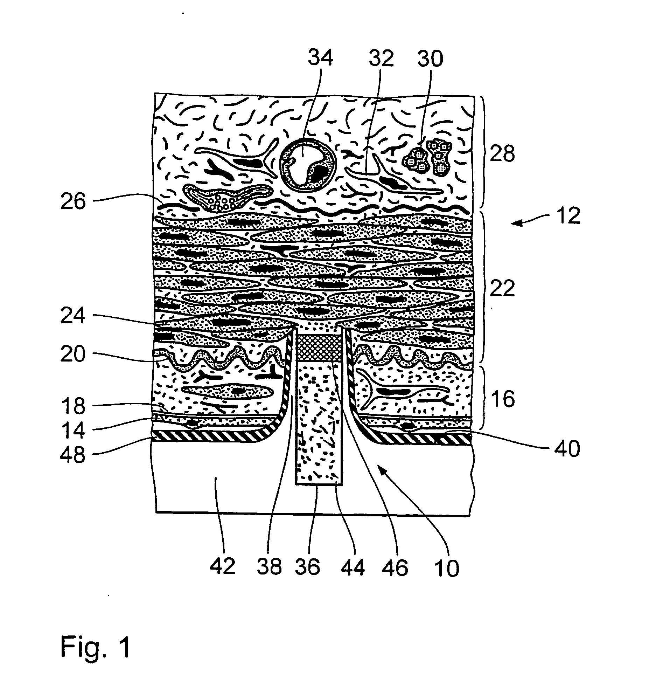

Die Fig. 1 zeigt in schematischer Weise einen Querschnitt durch ein endovaskuläres Implantat im Bereich einer Mikrovorrichtung 10, die bereits in eine Gefäßwand 12 einer muskulären Arterie eingedrungen ist. Das endovaskuläre Implantat kann insbesondere ein Stent sein. Der Stent wird aus einem biokompatiblen Material, beispielsweise Nitinol, medizinischen Stahl, Tantal, Platin-Iridium-Legierungen, Gold oder dergleichen, geformt. Denkbar ist auch, eine biodegradierbare Magnesiumlegierung einzusetzen. Design und Dimensionierung des Stents können in einem weiten Maße variabel ausgelegt sein. Sie müssen lediglich die Anordnung bzw. das Einbringen der Mikrovorrichtungen 10 auf ihrer Außenfläche zulassen.

Die Gefäßwand 12 der Arterie ist nach herrschender histologischer Lehre dreischichtig gegliedert. Den inneren Endothelzellen 14 folgend, die die Gefäßwand 12 auskleiden, erstreckt sich der Bereich der sogenannten Intima 16, der von einer Basallamina 18 und einer inneren elastischen Membran 20 begrenzt wird. Der Intima 16 schließt sich die Media 22 an, die bei muskulären Arterien aus einer Vielzahl von Myofibroblasten 24, also glatten Muskelzellen, gebildet wird. Durch eine äußere elastische Membran 26 von der Media 22 getrennt folgt die Adventitia 28. In der Adventitia 28 liegen Nervenfaserbündel 30, Fibroblasten 32 und Blutgefäße 34.

Bei Verletzungen der Gefäßwand 12 - sei es durch Ballondilatation oder auch bei der Fixierung eines Stents in der gewünschten Lage - können die Restenose verursachende Mechanismen ausgelöst werden. An den der Restenose zugrunde liegenden Prozessen sind insbesondere Media 22 und Adventitia 28 beteiligt, sei es als Initiator der Proliferation durch Freisetzung von Entzündungsmediatoren oder als Ursprungsort der Zellmigration bei der Neointima-Proliferation, bei der die Myofibroblasten 24 im der Blutbahn zugewandtem Bereich der Intima 16 fibröse Deckschichten ausbilden. Eine wirksame Bekämpfung der Restenose muss also insbesondere in diesen Bereichen stattfinden.

Zu diesem Zwecke weist der Stent die Mikrovorrichtung 10 auf, die durch die Intima 16 bis in die Media 22 ragt und dort in noch näher erläuterter Weise einen therapeutischen Wirkstoff freisetzen kann. Die Mikrovorrichtung 10 besteht aus einem Wirkstoffdepot 36 und wenigstens einer verbundenen Mikrokanüle 38. Beide Komponenten können, wie hier dargestellt, ineinander übergehen. Um tatsächlich auch den Ort des Wirkens, nämlich die Media 22, zu erreichen, ragt die Mikrokanüle 38 100 bis 400 um aus einer der Gefäßwand 12 zugewandten Oberfläche 40 des Stents hervor. Für den intrakardialen Einsatz haben sich Mikrokanüllängen von 150 bis 300 µm, insbesondere 180 bis 250 µm als besonders effektiv erwiesen, da sie in weit über 90% der Fälle die gewünschte Lage einnehmen können. Die Länge muss so ausgelegt sein, dass auch eventuell vorhandener Plaque in der Intima 16 durchdrungen werden kann. Ein Durchmesser der Mikrokanüle 38 liegt bei etwa 20 bis 200 µm.

Die Anzahl und Lage der Mikrovorrichtungen 10 auf dem Stent kann den jeweiligen Erfordernissen angepasst werden und ist im wesentlichen abhängig von der Menge des zu eluierenden Wirkstoffs und vor allem dem Stent-Design. In der Regel soll eine möglichst großflächige und homogene Verteilung der Mikrovorrichtungen 10 auf Oberfläche 40 erzielt werden, um eine gleichmäßige Dosierung des Wirkstoffs sicherzustellen. Da die Diffusionsvorgänge in der Media 22 relativ langsam ablaufen, sollte die Anzahl der Mikrovorrichtungen 10 auf der Umfangsfläche des Stents etwa in der Größenordung von 2 bis 20 pro cm² liegen.

Das Wirkstoffdepot 36 und die Mikrokanüle 38 sind im vorliegenden Beispiel vollständig in einen Grundkörper 42 des Stents eingearbeitet. Denkbar ist auch, die Mikrovorrichtung 10 in Hybrid-Technik, also als vom Grundkörper 42 abgegrenzte Struktur, zu realisieren. Zwei Möglichkeiten, wie die Herstellung in der Praxis erfolgen kann, werden weiter unter noch näher erläutert.

In das Wirkstoffdepot 36 der Mikrovorrichtung 10 wird ein Wirkstoff eingebracht-hier angedeutet durch eine Schicht 44. Durch eine Deckschicht 46 aus einem biodegradierbarem Material bleibt das Wirkstoffdepot 36 zunächst verschlossen. Ein Degradationsverhalten der Deckschicht 46 wird so festgelegt, dass eine Freisetzung des Wirkstoffs erst nach dem vollständigen Eindringen in die Media 22 stattfinden kann. Die Oberfläche 40 des Stents ist weiterhin mit einer ebenfalls biodegradierbaren Zusatzschicht 48 bedeckt, deren Funktion weiter unten noch näher erläutert wird.

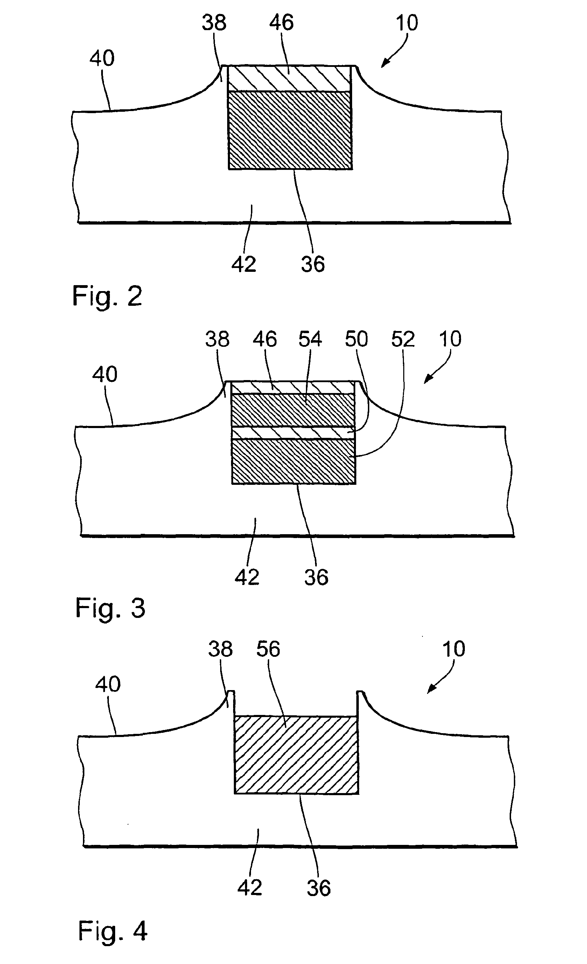

Der Fig. 2 ist eine erste Variante zu entnehmen, wie das Freisetzungsverhalten des Wirkstoffs in der gewünschten Weise beeinflusst werden kann. Dazu wird der Wirkstoff in das Wirkstoffdepot 36 in seiner pharmazeutischen bevorzugten Applikationsform eingebracht. Anschließend wird das Wirkstoffdepot 36 durch die bioresorbierbare Deckschicht 46 verschlossen.

Sollen mehrere Wirkstoffe nacheinander freigesetzt werden, so kann - wie in Fig. 3 schematisch angedeutet - neben der Deckschicht 46 eine weitere Trennschicht 50 im Wirkstoffdepot 36 vorhanden sein. Die Trennschicht 50 teilt das Volumen des Wirkstoffdepots 36 in zwei Bereiche 52, 54 auf, in denen jeweils ein Wirkstoff in seiner pharmazeutisch gewünschten Applikationsform bereitgestellt wird.

**Fig. 4** zeigt eine weitere, alternative Ausführungsform zur Lösung des Freisetzungsproblems. Der Wirkstoff wird in einem geeigneten Arzneistoffträger eingebettet und als homogene, bioresorbierbare Schicht 56 in das Wirkstoffdepot 36 eingebracht. Durch allmählichen Abbau der bioresorbierbaren Schicht 56 wird der Wirkstoff allmählich freigesetzt.

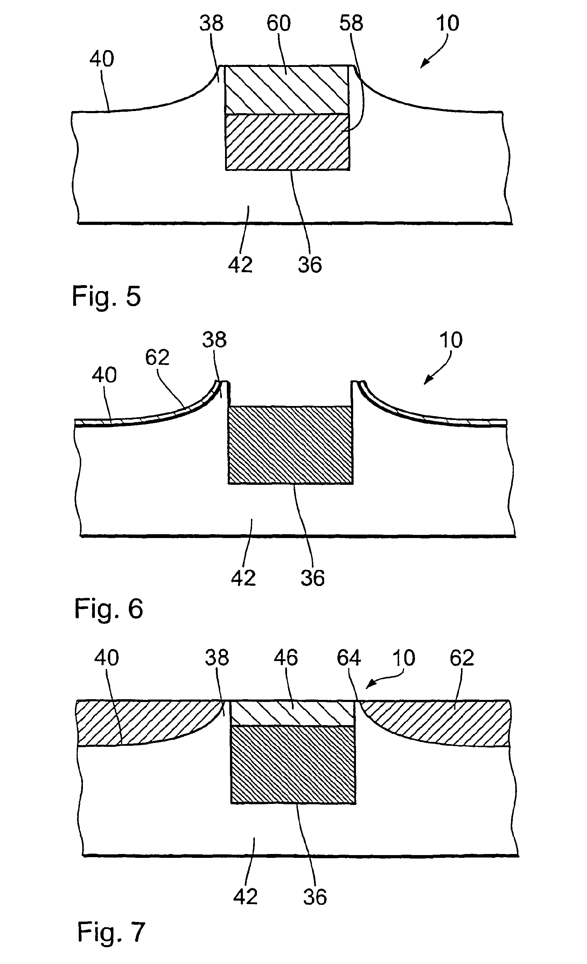

Nach einer weiteren Variante können auch mehrere Schichten aus bioresorbierbaren Arzneistoffträgern mit gegebenenfalls verschiedenen Wirkstoffen in das Wirkstoffdepot 36 eingebracht werden. In der Fig. 5 sind beispielhaft zwei derartige Schichten 58 und 60 angedeutet. Die Schicht 58 enthält einen Wirkstoff, der erst zu einem späteren Zeitpunkt in die Media 22 appliziert werden soll, während die obere Schicht 60 einen Wirkstoff beinhaltet, der möglichst frühzeitig die Restenose auslösende Mechanismen hemmen soll. In diesem Sinne könnte beispielsweise ein die Freisetzung von Entzündungsmediatoren hindernder erster Wirkstoff in die Schicht 60 und ein die Zellmigration beeinflussender zweiter Wirkstoff in die Schicht 58 eingesetzt werden. Denkbar ist auch, durch Einbringung mehrerer Schichten eine temporäre Dosis eines einzelnen Wirkstoffs oder einer Wirkstoffkombination festzulegen, indem die Schichten unterschiedliche Gehalte des Wirkstoffs bzw. der Wirkstoffkombination aufweisen. Ebenso kann eine Variation des Degradationsverhaltens der einzelnen Schichten im Wirkstoffdepot zur Beeinflussung der temporären Dosis genutzt werden.

In der Fig. 6 ist schematisch eine weitere, alternative Form der Bereitstellung des Wirkstoff in der Mikrovorrichtung 10 dargestellt, bei der die Oberfläche 40 des Stents mit einer Zusatzschicht 62 aus einem bioresorbierbaren Material bedeckt ist. Die Zusatzschicht 62 dient der Unterdrückung entzündlicher Prozesse und kann, wie in Fig. 7 dargestellt, bündig an eine obere Spitze 64 der Mikrokanüle 38 anschließen oder gar die gesamte Mikrovorrichtung 10 vollständig abdecken. Dadurch wird erreicht, dass der Stent beim Implantieren nicht durch die Mikrovorrichtungen 10 die Gefäßwand 12 verletzt bzw. die Mikrovorrichtungen 10 mechanische beschädigt werden (protective covering).

Mit dem allmählichen Abbau der Zusatzschicht 62 dringt die Mikrokanüle 38 durch die Intima 16 bis in die Media 22 ein. Dieser Vorgang kann durch selbstexpandierende Strukturen im Stent unterstützt werden. Beispielsweise kann dazu eine Gedächtnislegierung wie Nitinol verwendet werden. Aber auch eine manuelle Dilatation mit Ballonkathetern in bestimmten Zeitabständen ist denkbar. Um eine frühzeitige Freisetzung des Wirkstoffs zu verhindern, wird das Degradationsverhalten der Deckschicht 46 so abgestimmt, dass deren Abbau erst nach vollständigem Abbau der Zusatzschicht 62 abgeschlossen ist und damit die Mikrokanüle 38 aller Wahrscheinlichkeit nach bereits den Wirkort, nämlich die Media 22 der Gefäßwand 12, erreicht hat.

Als bioresorbierbare Materialien für die genannten Deckschichten, Trennschichten, Zusatzschichten und Arzneistoffträger eignen sich insbesondere Polycaprolactone (PCL), Poly-D,L-lactidsäuren (DL-PLA), Poly-L-lactidsäuren (L-PLA), Polyhydroxybutyrate, Polydioxanone, glycolidische Polylactide, Polyorthoester, Polyanhydride, Polyglycolsäuren und deren Copolymere, Polyphosphorester, Polyaminosäuren, Polytrimethylencarbonat, Polyphosphazene, Polyiminocarbonate, aliphatische Polycarbonate, Heparin, Fibrin, Fibrinogen, Cellulose, Kollagen, Alginate, Chitosane und vernetzte Hyaluronsäuren.

Wirkstoffe zur direkten Applikation in die Media 22 sind insbesondere anti-angiogene, anti-inflammatorische und anti-proliferative therapeutisch-wirksame Substanzen. Die anti-angiogene Substanzen umfassen beispielsweise Retinonsäure und ihre Derivate, Suramin, Metallproteinasen-1- und Metallproteinasen-2-Inhibitoren, Epothilon, Colchicin, Vinblastin und Paclitaxel. Anti-inflammatorische Substanzen umfassen z.B. Salicylate, Fenoprofen, Ketoprofen, Tolmetin, Kortison, Dexamethason, Cyclosporin, Azatidin, Rapamycin, Tacrolimus, Everolimus und Diphenylimidazol. Als anti-proliferative therapeutische Substanzen kommen insbesondere Antiestrogene und Hormone (Estradiol, Tamoxifen, Testolacton, etc.) und cytotoxische Mittel (Bleomycin, Doxorubizin, ldarubicin, Colchicin, etc.) in Frage.

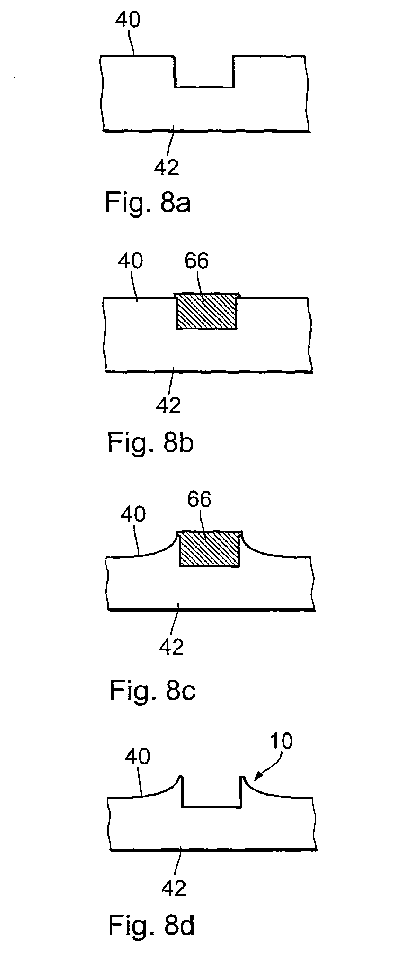

Die Fig. 8a bis 8d zeigen in schematischer Weise einen Bereichsausschnitt aus einem Stent in dem eine Mikrovorrichtung 10 durch bestimmte Bearbeitungsschritte erzeugt werden soll. Die einzelnen Strukturelemente des metallischen Grundkörpers 42 des Stents werden üblicherweise durch Laserschneiden erzeugt. Geeignet sind beispielsweise Laser mit einem Arbeitsbereich um 1000 nm und einer Leistung von etwa 3-5 W, deren Pulslänge im Bereich weniger hunderstel Millisekunden variiert werden kann und die auf einen Bereich weniger µm² fokussierbar sind. Vor, während oder im Anschluss an das Schneiden der Stege etc. kann durch gezielte partielle Laserabtragung eine Kavität in die Oberfläche 40 des Grundkörpers 42 eingebracht werden (siehe Fig. 8a). Es versteht sich von selbst, dass die Arbeitsparameter des Lasers während der partiellen Laserabtragung gegenüber den Arbeitsparametern während des Laserschneidens verändert sind. Sinnvollerweise ist die Leistung, die Pulsdauer und/oder die Wechselwirkungsdauer herabgesetzt. Die Tiefe der Kavität soll dabei zumindest der gewünschten Länge der Mikrokanüle entsprechen, also bei mindestens 100 µm liegen. Ebenso wird durch den Durchmesser der erzeugten Kavität ein Durchmesser der Mikrokanüle vorgegeben (dieser liegt üblicherweise bei 20 bis 200 µm).

In einem sich anschließenden Verfahrensschritt (Fig. 8b) wird die Kavität und leicht überlappend auch ein die Kavität umlaufender Rand mit einem elektrisch isolierenden Material 66 abgedeckt. Als Material 66 kommen beispielsweise Lack, Wachs oder ähnliches in Frage, welches als Emulsion oder Lösung durch geeignete Sprühtechniken aufgebracht wird. Durch das sich anschließende Elektropolieren wird der Grundkörper 42 abgetragen, wobei in den vom isolierenden Material 66 bedeckten Bereichen der Oberfläche 40 der Grundkörper 42 stehen bleibt und allmählich die Mikrokanüle ausbildet (Fig. 8c). Auch das Elektropolieren wird routinemäßig bei der Herstellung von Stents angewandt, da erwiesenermaßen glatte Oberflächen die Restenoserate weiter senken. Zuletzt wird das isolierende Material 66 mit geeigneten Lösungsmitteln wieder entfernt (Fig. 8d).

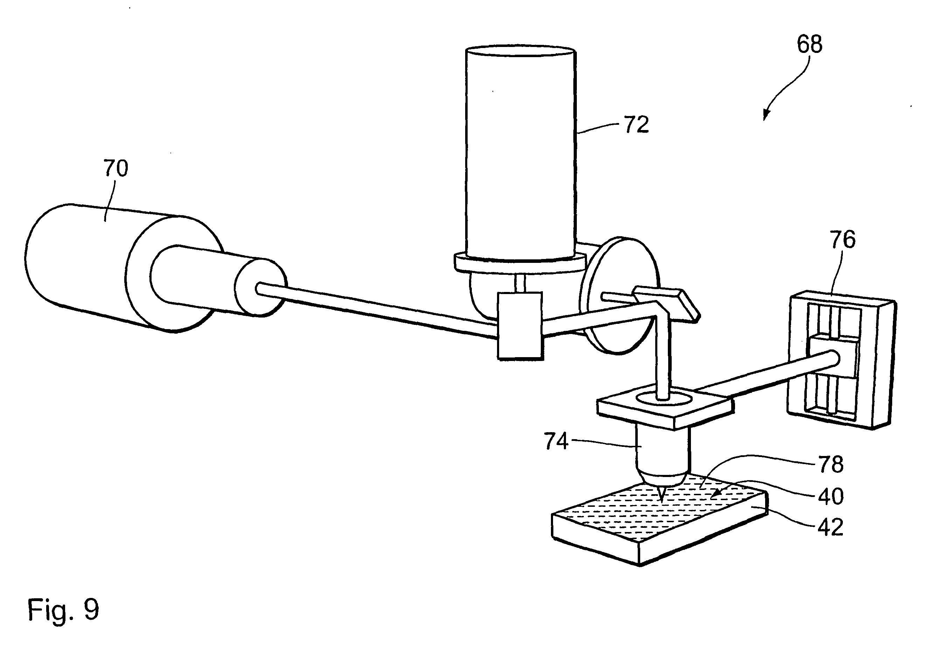

Statt einer vollständigen Integration der Mikrovorrichtung 10 in den Grundkörper 42 des Stents, wie sie vorhergehend beschrieben wurde, kann diese auch in Hybrid-Technik als separate Struktur auf die Oberfläche 40 des Implantats aufgebracht werden. Für derartige Mikrostrukturen wurden mittlerweile Rapid-Prototyping-Verfahren basierend auf Mikrolithographieschritten entwickelt (siehe z.B. TÖNS-HOFF, H.K.; KÖRBER, K.; BEIL, A.: Micro-rapid prototyping - a new machine system enabling micro-stereolithography and laser sintering processes. In: Proc. of Ml-CRO.tec 2000, Vol. 2, 25.-27. Sept. 2000, Hannover. - ISBN 3-8007-2579-7, Seite 37-42). Hierbei werden geeignete Harze durch einen möglichst schmalen, fokussierten Laserstrahl im belichteten Bereich zur Polymerisation gebracht. Neben dem Einsatz von Polymeren können auch Sinterwerkstoffe Verwendung finden. Fig. 9 zeigt vereinfacht den prinzipiellen Aufbau einer zur Durchführung des Rapid-Prototyping-Verfahrens geeigneten Mikro-Stereolithographie-Vorrichtung 68.

Die Mikrovorrichtungen 10 werden Schicht für Schicht durch Polymerisation ausgehend.von der Oberfläche 40 des Grundkörpers 42 aufgebaut. Eine Laserquelle 70 dient als gepulste Lichtquelle. Über eine Scanner-Einheit 72 wird der erzeugte Laserstrahl in ein stark fokussierendes Objektiv 74, das über ein Stellglied 76 beweglich gelagert ist, eingespeist. Die Oberfläche 40 des Grundkörpers 42 ist mit einer wenige Mikrometer dünnen, flüssigen Monomerschicht 78 bedeckt. Der Laserstrahl belichtet schraffurartig eine definierte Fläche auf der Oberfläche 40 im flüssigen Monomer und härtet auf diese Weise mit einer bestimmten Eindringtiefe im Fokusbereich eine Schicht des zu fertigenden Modells aus. Das Verstellglied 76 sorgt dafür, dass das Substrat bzw. den Laserfokus Schicht um Schicht um die definierte Schichtdicke, üblicherweise in Mikrometerschritten, abgesenkt bzw. hochgefahren wird. Im folgenden Bearbeitungsschritt wird das Monomer über der zuvor erzeugten festen Schicht durch Belichtung (durch Ein- bzw. Multiphotonenprozesse) zur Polymerisation gebracht, und dieser Bearbeitungsschritt wird solange wiederholt bis die gewünschte Struktur steht. Nach der Schichtengenerierung werden verbleibende Monomerreste durch geeignete Lösungsmittel entfernt. Eine komplexe Steuerung (ebenfalls nicht dargestellt) überwacht die Herstellung über die Scanner-Einheit 72. Die Steuerung koordiniert anhand eines z.B. in CAD-Format erstellten Modells die Bewegungsabläufe des Objektivs 74, die Intensität und Pulsdauer der Laserquelle 70 und die Bewegung des Fahrstuhls. Da Mikro-Lithographie-Vorrichtungen 68 der dargestellten Art bereits hinreichend aus dem Stand der Technik bekannt sind, wird auf eine detaillierte Beschreibung verzichtet.

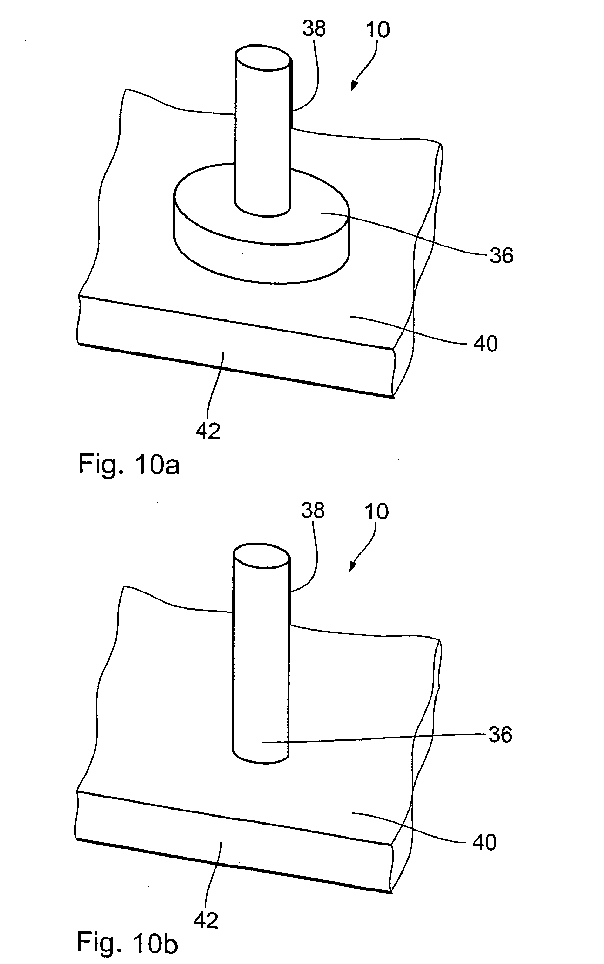

Eine erste durch Rapid-Prototyping-Verfahren erzeugte Mikrovorrichtung 10 zeigt schematisch die Figur 10a. Die Mikrovorrichtung 10 beinhalt eine röhrenförmige Mikrokanüle 38, die auf einem zylindrischen Wirkstoffdepot 36 aufgebracht ist. Die Gesamthöhe der Struktur beträgt etwa 200 µm. Die beiden Hauptbestandteile der Mikrovorrichtung 10 können auch - wie in Fig. 10b dargestellt - konturlos ineinander übergehen. Die röhrenförmige Mikrovorrichtung 10 ragt etwa 150 µm von der Oberfläche 40 des Grundkörpers 42 empor. Vorzugsweise werden biokompatible Polymere für die Herstellung verwendet.

Eine Einbringung des Wirkstoffs in die bereitgestellten Mikrovorrichtungen 10 kann beispielsweise derart erfolgen, dass die Stentoberfläche 40 mit einer Lösung des Wirkstoffs in einem geeigneten Lösungsmittel benetzt wird, wobei die Lösung auch in die Mikrovorrichtungen 10 eindringt. Nach dem Trocknen wird der Wirkstoff in den Bereichen außerhalb der Mikrovorrichtungen 10 abgeblasen. Dazu kann beispielsweise eine Luftlanze mit einigen kPa Gebläseleistung in einem Winkel von etwa 90° und einem Abstand von einigen Zentimetern eingesetzt werden. In gleicher Weise kann auch bei der Aufbringung der Polymere für Deckschichten, Trennschichten und Arzneistoffträger vorgegangen werden. Abschließend kann die Oberfläche 40 mit einer schützenden Zusatzschicht, z.B. im Tauch- oder Sprühverfahren, bedeckt werden (protective covering).

## Patentansprüche

1. Endovaskuläres Implantat zur Applikation eines Wirkstoffs in die Media (22) eines Blutgefäßes, mit einem Grundkörper (42), der zumindest abschnittsweise an einer dem Blutgefäß zugewandten Oberfläche (40) des Implantats eine Vielzahl von Mikrovorrichtungen (10) zur Applikation des Wirkstoffs aufweist, wobei jede Mikrovorrichtung (10)
- zumindest eine Mikrokanüle (38), die eine Länge von 100-400 µm aufweist und damit soweit aus der Oberfläche des Implantats herausgehoben ist, dass sie beim flächigen Anlegen des Implantats an eine Gefäßwand (12) des Blutgefäßes bis in die Media (22) des Blutgefäßes eindringt und
- zumindest ein Wirkstoffdepot (36), dass mit zumindest einer Mikrokanüle (38) in Verbindung steht, umfasst und
wobei das Implantat ein Stent ist.

2. Implantat nach Anspruch 1, **dadurch gekennzeichnet, dass** die Mikrokanülen (38) eine Länge von 150-300 µm aufweisen.

3. Implantat nach Anspruch 2, **dadurch gekennzeichnet, dass** die Mikrokanülen (38) eine Länge von 180-250 µm aufweisen.

4. Implantat nach einem der vorhergehenden Ansprüche, **dadurch gekennzeichnet, dass** die Mikrokanülen (38) einen Durchmesser von 20-200 µm haben.

5. Implantat nach einen der vorhergehenden Ansprüche, **dadurch gekennzeichnet, dass** die Mikrovorrichtungen (10) Bestandteile eines Grundkörpers (42) des Implantats sind.

6. Implantat nach einen der vorhergehenden Ansprüche, **dadurch gekennzeichnet, dass** die Mikrovorrichtungen (10) in Hybrid-Technik auf den Grundkörper (42) des Implantats aufgebracht sind.

7. Implantat nach einen der vorhergehenden Ansprüche, **dadurch gekennzeichnet, dass** ein Freisetzungsverhalten des zumindest einen deponierenden Wirkstoffs so festgelegt ist, dass der zumindest eine Wirkstoff erst nach Eindringen der Mikrokanülen (38) in die Media (22) des Blutgefäßes freigesetzt wird.

8. Implantat nach Anspruch 7, **dadurch gekennzeichnet, dass** die Mikrovorrichtung (10) nach Einbringung des zumindest einen Wirkstoffs in das Wirkstoffdepot (36) durch eine Deckschicht (46) aus einem biodegradierbaren Material verschlossen ist.

9. Implantat nach Anspruch 7 oder 8, **dadurch gekennzeichnet, dass** der zumindest eine Wirkstoff in einem biodegradierbaren Arzneistoffträger eingebettet ist.

10. Implantat nach einem der Ansprüche 7 bis 9, **dadurch gekennzeichnet, dass** mehrere Wirkstoffe so in das Wirkstoffdepot (36) eingebracht sind, dass eine stufenweise Freisetzung der Wirkstoffe erfolgt.

11. Implantat nach Anspruch 10, **dadurch gekennzeichnet, dass** mehrere Schichten (58, 60) biodegradierbarer Arzneistoffträger mit eingebetteten Wirkstoffen in das Wirkstoffdepot (36) eingebracht sind, die nacheinander abgebaut werden.

12. Implantat nach Anspruch 10, **dadurch gekennzeichnet, dass** eine oder mehrere Trennschichten (50) aus einem biodegradierbaren Material vorhanden sind, die nacheinander abgebaut werden und die verschiedenen Wirkstoffe voneinander trennen.

13. Implantat nach einem der vorhergehenden Ansprüche, **dadurch gekennzeichnet, dass** die außerhalb der Mikrovorrichtung (10) liegenden Bereiche der Oberfläche (40) des Implantats mit einer Schicht (62) aus einem biodegradierbarem Material bedeckt sind.

14. Implantat nach Anspruch 13, **dadurch gekennzeichnet, dass** die Schicht (62) in Umfangsrichtung bündig an einer Spitze (64) der Mikrokanülen (38) der Mikrovorrichtung (10) abschließt oder die Mikrovorrichtung (10) vollständig bedeckt und ein Abbauverhalten der Schicht (62) mit dem Freisetzungsverhalten des Wirkstoffs abgestimmt ist, dass die Freisetzung des Wirkstoffs erst nach dem vollständigen Abbau der Schicht (62) beginnt.

15. Implantat nach Anspruch 14, **dadurch gekennzeichnet, dass** im Implantat selbstexpandierende Strukturen vorhanden sind, die ein allmähliches Eindringen der Mikrokanülen (38) in die Gefäßwand (12) unterstützen.

16. Implantat nach den Ansprüchen 13 bis 15, **dadurch gekennzeichnet, dass** die Schicht (62) aus Hyaluronsäurepolymeren mit differenter Degradationskinetik ist.

17. Implantat nach einem der vorhergehenden Ansprüche, **dadurch gekennzeichnet, dass** das Implantat ein Koronarstent, ist.

18. Implantat nach Anspruch 17, **dadurch gekennzeichnet, dass** der Grundkörper (42) des Stents zumindest abschnittsweise aus einem biodegradierbaren Material, insbesondere einer Magnesiumlegierung, geformt ist.

19. Verfahren zur Herstellung eines Implantats gemäß eines der Ansprüche 1 bis 18 mit wenigstens einer Mikrovorrichtung (10) zur Injektion eines Wirkstoffs in die Media (22) eines Blutgefäßes, bei dem auf dem metallischen, medizinischen Implantat
a) eine Kavität auf einer der Gefäßwand zugewandten Oberfläche (40) des Implantats durch partielle Materialabtragung, insbesondere Laserabtragung, erzeugt wird,
b) die Kavität und ein umlaufender Rand der Kavität mit einem isolierenden Material (66) bedeckt werden,
c) durch Elektropolieren eine Materialabtragung in den nicht durch das isolierende Material (66) bedeckten Bereichen der Oberfläche (40) erfolgt und
d) das isolierende Material (66) durch geeignete Lösungsmittel entfernt wird.

## Claims

1. An endovascular implant for administration of an agent into the media (22) of a blood vessel, having a main body (42), which has a plurality of microdevices (10) for the administration of the agent at least sectionally on the surface (40) of the implant facing toward the blood vessel, wherein each microdevice (10) comprises
- at least one microcannula (38), which has a length of 100-400 µm and thus protrudes far enough out of the surface of the implant that it penetrates up into the media (22) of the blood vessel when the implant lies flatly against a vascular wall (12) of the blood vessel and
- at least one agent depot (36), which is connected to at least one microcannula (38), and
wherein the implant is a stent.

2. The implant according to Claim 1, **characterized in that** the microcannulas (38) have a length of 150-300 µm.

3. The implant according to Claim 2, **characterized in that** the microcannulas (38) have a length of 180-250 µm.

4. The implant according to one of the preceding claims, **characterized in that** the microcannulas (38) have a diameter of 20-200 µm.

5. The implant according to one of the preceding claims, **characterized in that** the microdevices (10) are components of a main body (42) of the implant.

6. The implant according to one of the preceding claims, **characterized in that** the microdevices (10) are applied in hybrid technology to the main body (42) of the implant.

7. The implant according to one of the preceding claims, **characterized in that** a release behavior of the at least one deposited agent is established in such a manner that the least one agent is only released after penetration of the microcannulas (30) into the media (22) of the blood vessel.

8. The implant according to Claim 7, **characterized in that** the microdevice (10) is closed by a cover layer (46) made of a biodegradable material after introduction of the at least one agent into the agent depot (36).

9. The implant according to Claim 7 or 8, **characterized in that** the at least one agent is embedded in a biodegradable pharmaceutical carrier.

10. The implant according to one of Claims 7 through 9, **characterized in that** multiple agents are introduced into the agent depot (36) in such a manner that a step-by-step release of the agents occurs.

11. The implant according to Claim 10, **characterized in that** multiple layers (58, 60) of biodegradable pharmaceutical carriers having embedded agents are introduced into the agent depot (36), which are degraded in sequence.

12. The implant according to Claim 10, **characterized in that** one or more partition layers (50) made of a biodegradable material are provided, which are degraded in sequence and separate the various agents from one another.

13. The implant according to one of the preceding claims, **characterized in that** the areas of the surface (40) of the implant lying outside the microdevice (10) are covered by a layer (62) made of a biodegradable material.

14. The implant according to Claim 13, **characterized in that** the layer (62) terminates flush with a tip (64) of the microcannulas (38) of the microdevice (10) in the peripheral direction or completely covers the microdevice (10) and a degradation behavior of the layer (62) is adapted to the release behavior of the agent in such a manner that the release of the agent only begins after the complete degradation of the layer (62).

15. The implant according to Claim 14, **characterized in that** self-expanding structures are provided in the implant, which support a gradual penetration of the microcannulas (38) into the vascular wall (12).

16. The implant according to Claims 13 through 15, **characterized in that** the layer (62) is made of hyaluronic acid polymers having different degradation kinetics.

17. The implant according to one of the preceding claims, **characterized in that** the implant is a coronary stent.

18. The implant according to Claim 17, **characterized in that** the main body (42) of the stent is at least sectionally formed from a biodegradable material, in particular a magnesium alloy.

19. A method for producing an implant according to one of Claims 1 through 18 having at least one microdevice (10) for injecting an agent into the media (22) of a blood vessel, in which, on the metallic medical implant,
a) a cavity is generated on a surface (40) of the implant facing toward the vascular wall by partial material ablation, in particular laser ablation,
b) the cavity and a peripheral edge of the cavity are covered using an insulating material (66),
c) a material ablation is performed by electropolishing in the areas of the surface (40) not covered by the insulating material (66), and
d) the insulating material (66) is removed by suitable solvents.

## Revendications

1. Implant endovasculaire pour l'application d'un agent actif dans la tunique médiane (22) d'un vaisseau sanguin, comprenant un corps de base (42), qui présente une pluralité de microdispositifs (10) pour l'application de l'agent actif au moins par tronçons sur une surface (40), tournée vers le vaisseau sanguin, de l'implant, chaque microdispositif (10) comportant
- au moins une microcanule (38), qui présente une longueur de 100 à 400 µm et est ainsi soulevée de la surface de l'implant, de telle sorte que, lors de l'application à plat de l'implant sur une paroi de vaisseau (12) du vaisseau sanguin, elle pénètre jusque dans la tunique médiane (22) du vaisseau sanguin et,
- au moins un dépôt d'agent actif (36), qui est en liaison avec au moins une microcanule (38), et
l'implant étant un stent.

2. Implant selon la revendication 1, **caractérisé en ce que** les microcanules présentent une longueur de 150 à 300 µm.

3. Implant selon la revendication 2, **caractérisé en ce que** les microcanules (38) présentent une longueur de 180 à 250 µm.

4. Implant selon l'une quelconque des revendications précédentes, **caractérisé en ce que** les microcanules (38) présentent un diamètre de 20 à 200 µm.

5. Implant selon l'une quelconque des revendications précédentes, **caractérisé en ce que** les microdispositifs (10) sont des composants d'un corps de base (42).

6. Implant selon l'une quelconque des revendications précédentes, **caractérisé en ce que** les microdispositifs (10) sont appliqués dans la technique hybride sur le corps de base (42) de l'implant.

7. Implant selon l'une quelconque des revendications précédentes, **caractérisé en ce qu'**un comportement à la libération du au moins un agent actif qui se dépose est fixé de telle sorte que le au moins un agent actif n'est libéré qu'après la pénétration des microcanules (38) dans la tunique médiane (22) du vaisseau sanguin.

8. Implant selon la revendication 7, **caractérisé en ce que** le microdispositif (10) est fermé, après l'introduction du moins un agent actif dans le dépôt d'agent actif (36), par une couche de recouvrement (46) à base d'un matériau biodégradable.

9. Implant selon la revendication 7 ou 8, **caractérisé en ce que** le au moins un agent actif est incorporé dans un support de médicament biodégradable.

10. Implant selon l'une quelconque des revendications 7 à 9, **caractérisé en ce que** plusieurs agents actifs sont introduits dans le dépôt d'agent actif (36) de telle sorte qu'on a une libération progressive des agents actifs.

11. Implant selon la revendication 10, **caractérisé en ce que** plusieurs couches (58, 60) de supports de médicament biodégradables avec des agents actifs incorporés sont introduites dans le dépôt d'agent actif (36), lesquels sont dégradés les uns après les autres.

12. Implant selon la revendication 10, **caractérisé en ce qu'**une ou plusieurs couches de séparation (50) à base d'un matériau biodégradable sont présentes, lesquelles sont dégradées de façon successive et séparent les différents agents actifs les uns des autres.

13. Implant selon l'une quelconque des revendications précédentes, **caractérisé en ce que** les zones, situées à l'extérieur du microdispositif (10), de la surface (40) de l'implant sont recouvertes d'une couche (62) à base d'un matériau biodégradable.

14. Implant selon la revendication 13, **caractérisé en ce que** la couche (62) arrive dans le sens périphérique au niveau d'une pointe (64) des microcanules (38) du microdispositif (10) ou bien couvre complètement le microdispositif (10) ou bien un comportement à la dégradation de la couche (62) est assorti au comportement à la libération de l'agent actif, de telle sorte que la libération de l'agent actif ne commence qu'après la dégradation complète de la couche (62).

15. Implant selon la revendication 14, **caractérisé en ce que** des structures autoexpansibles, qui favorisent une pénétration progressive des microcanules (38) dans la paroi de vaisseau (12), sont présentes dans l'implant.

16. Implant selon les revendications 13 à 15, **caractérisé en ce que** la couche (62) est à base de polymères d'acide hyaluronique avec une cinétique de dégradation différente.

17. Implant selon l'une quelconque des revendications précédentes, **caractérisé en ce que** l'implant est un stent coronaire.

18. Implant selon la revendication 17, **caractérisé en ce que** le corps de base (42) du stent est formé au moins par portions à base d'un matériau biodégradable, en particulier un alliage de magnésium.

19. Procédé de fabrication d'un implant selon l'une des revendications 1 à 18, comprenant au moins un microdispositif (10) pour l'injection d'un agent actif dans la tunique médiane (22) d'un vaisseau sanguin, dans lequel, sur l'implant médical métallique,
a) on génère une cavité sur une surface (40), tournée vers la paroi de vaisseau, de l'implant par enlèvement de matériau partiel, en particulier enlèvement au laser,
b) la cavité et un bord périphérique de la cavité sont recouverts avec un matériau (66) isolant,
c) un enlèvement de matériau s'effectue par électropolissage dans les zones de la surface (40) qui ne sont pas recouvertes par le matériau (66) isolant et
d) le matériau (66) isolant est enlevé par des solvants appropriés.
